# EUROPEAN PATENT SPECIFICATION

(11) **EP 1 965 698 B1**
(45) Date of publication and mention of the grant of the patent: **19.02.2014**
(21) Application number: 06821665.4
(22) Date of filing: 26.12.2006
(51) Int. Cl.: A61B 5/05, A61B 5/06

(54) **SYSTEM AND METHOD OF IN-VIVO MAGNETIC POSITION DETERMINATION**
SYSTEM UND VERFAHREN FÜR DIE MAGNETPOSITIONSBESTIMMUNG IN VIVO
SYSTEME ET PROCEDE DE DETERMINATION DE POSITION MAGNETIQUE IN VIVO

(30) Priority: 29.12.2005 US 754288 P
(43) Date of publication of application: 10.09.2008
(73) Proprietor: Given Imaging Ltd., 20692 Yoqneam (IL)
(72) Inventor: BETTESH, Ido, Zichron Ya'akov, 30900 (IL)
(74) Representative: Dr. Graf & Partner AG
(86) International application number: PCT/IL2006/001483
(87) International publication number: WO 2007/074445

(56) References cited:
- EP-A1- 1 374 793
- EP-A1- 1 790 279
- EP-A2- 0 894 473
- WO-A1-2006/030773
- US-A- 5 676 673
- US-A1- 2003 160 721
- US-A1- 2003 195 415
- US-B1- 6 233 476

## Description

### FIELD OF THE INVENTION

The present invention relates to the field of in-vivo sensing. More specifically, the present invention relates to devices, systems and methods for performing in-vivo magnetic position detection.

### BACKGROUND OF THE INVENTION

Devices, systems and methods for in-vivo sensing of passages or cavities within a body, and for sensing and gathering information (e.g., image information, pH information, temperature information, electrical impedance information, pressure information, etc.), are known in the art.

In-vivo sensing devices such as capsules may include a sensing system and a transmission system, wherein the sensing system collects data and the transmission system transmits the collected data using Radio Frequency (RF) electromagnetic energy to an external receiver system, e.g., for further processing and display.

Some in-vivo imaging systems include an image sensor carried within a swallowable device such as a capsule. The in-vivo imaging device may capture and transmit images of the gastrointestinal (GI) tract, or another body lumen or body cavity being imaged, while the device may pass through the entire digestive tract and may operate as an autonomous video endoscope.

Prior attempts have been made at tracking an intra-gastric and intrauterine transmitting device include spatially scanning a non-ambulatory patient with a receiver. The receiver and scanning system may locate the points with the highest reception and track the device, the assumption being that the device may be at the location where the strongest signal may have been received. Such systems may require laboratory equipment that may not be portable and may not be commercial.

EP 1 790 279 and US 6 233 476 disclose a capsule endoscope comprising an image detector, a magnetic sensor for detecting an external magnetic field, an antenna for transmitting detected images and detected magnetic field parameters in order to evaluate the position of the capsule.

EP 1 374 793 deals with detecting the position of an implanted marker using an external magnetic field. The marker has no imaging device.

EP 894 473 and US 2003/160721 relate to determining the position of a catheter or endoscope using an external magnetic field. Images detected by the catheter are not transmitted by an antenna to an external device.

### SUMMARY OF THE INVENTION

Embodiments of the present invention are defined in the claims.

The in-vivo magnetic position detection device is autonomous. The in-vivo magnetic position detection device is a swallowable capsule which may be swallowed and moved through the GI tract.

The in-vivo device includes an in-vivo magnetic unit which detects electro-magnetic fields.

The system includes one or more antennas to detect the magnetic fields and a processing unit to process, analyze and calculate the magnetic fields measurements.

The system includes a position detection unit to calculate the position or the location of the in vivo device based on the detectors location, and on magnetic field intensity measurements.

There is provided a method for determining the position of an in-vivo sensing device comprising the steps of:
providing the in-vivo sensing device with an antenna;
generating magnetic fields external to the in-vivo sensing device;
detecting the magnetic fields by the antenna and measuring magnetic field intensity values of the detected magnetic fields; and
determining the position of the in-vivo sensing device from the measured magnetic field intensity values.

The method comprises the further step of transmitting the measured magnetic field intensity values to an external unit and wherein the step of determining the position of the in-vivo sensing device is performed in the external unit.

The magnetic fields are generated by at least one set of three adjacent transmitting antennas.

The three transmitting antennas are mutually perpendicular.

The transmitting antennas are coils.

The position of the in-vivo device is determined from the difference in power induced in each coil by the magnetic fields.

In accordance with some embodiments, the magnetic fields generated by the three transmitting antennas are generated at different times.

In accordance with some embodiments, the magnetic fields generated by the three transmitting antennas are generated simultaneously with different frequencies

There is provided a system for determining the position of an in-vivo sensing device comprising:
a magnetic field generator;
an antenna for measuring magnetic field intensity values of magnetic fields generated by the magnetic field generator; and
a processor for determining the position of the in-vivo sensing device from the magnetic field intensity values.

The processor is located external to the in-vivo sensing device.

The magnetic field generator comprises a coil.

The magnetic field generator is located external to the in-vivo sensing device.

The magnetic field generator comprises at least one set of three mutually perpendicular coils.

The antenna for measuring magnetic field intensity values is included in the in-vivo sensing device.

In accordance with some embodiments, the antenna for measuring magnetic field intensity values comprises at least one set of three mutually perpendicular coils.

### BRIEF DESCRIPTION OF THE DRAWINGS

The principles and operation of the system, apparatus, and method according to the present invention may be better understood with reference to the drawings, and the following description, it being understood that these drawings are given for illustrative purposes only and are not meant to be limiting, wherein:
Figure 1 is a schematic block diagram of an in-vivo sensing system in accordance with an embodiment of the invention;
Figures 2A and 2B are schematic illustrations of a patient wearing an antenna array according to an embodiment of the invention;
Figure 2C is a schematic illustration of three mutually perpendicular coils;
Figure 3 is a schematic block diagram of a data recorder in accordance with an embodiment of the invention;
Figure 4 is a flow-chart of a method of position detection according to an embodiment of the invention; and
Figure 5 is a flow-chart of a method of position detection according to an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements throughout the serial views.

### DETAILED DESCRIPTION OF THE INVENTION

In the following detailed description, numerous specifc details are set forth in order to provide a thorough understanding of the present invention. However, it will be understood by those skilled in the art that the present invention may be practiced without these specific details. In other instances, well-known methods, procedures, and components have not been described in detail so as not to obscure the present invention.

Some embodiments of the present invention are directed to a typically swallowable in-vivo device, e.g., a typically swallowable in-vivo sensing or imaging device. Devices according to embodiments of the present invention may be similar to embodiments described in United States Patent Application Number 09/800,470, entitled "Device and System for In-vivo Imaging", filed on 8 March, 2001, published on November 1, 2001 as United States Patent Application Publication Number 2001/0035902, and/or in United States Patent Number 5,604,531 to Iddan et al., entitled "In-Vivo Video Camera System", and/or in United States Patent Application Number 10/046,541, filed on January 16, 2002, published on August 15, 2002 as United States Patent Application Publication Number 2002/0109774. An external receiver/recorder unit, a processor and a monitor, e.g., in a workstation, such as those described in the above publications, may be suitable for use with some embodiments of the present invention. Devices and systems as described herein may have other configurations and/or other sets of components. For example, embodiments of the present invention may be practiced using an endoscope, needle, stent, catheter, etc. Some in-vivo devices according to embodiments of the present invention may be capsule shaped, or may have other shapes, for example, a peanut shape or tubular, spherical, conical, or other suitable shapes.

Some embodiments of the present invention may include, for example, a typically swallowable in-vivo device. In other embodiments, an in-vivo device need not be swallowable and/or autonomous, and may have other shapes or configurations. Some embodiments may be used in various body lumens, for example, the GI tract, blood vessels, the urinary tract, the reproductive tract, or the like. In some embodiments, the in-vivo device may include a sensor, an imager, and/or other suitable components.

Embodiments of the in-vivo device are typically autonomous and are typically self-contained. For example, the in-vivo device may be or may include a capsule or other unit where all the components are substantially contained within a container, housing or shell, and where the in-vivo device does not require any wires or cables to, for example, receive power or transmit information. The in-vivo device may communicate with an external receiving and display system to provide display of data, control, or other functions and the receiver/recorder unit may communicate with the in-vivo device, for example, to control components of the in-vivo device. Power may be provided, for example, by an internal battery or a wireless power receiving system. Other embodiments may have other configurations and capabilities. For example, components may be distributed over multiple sites or units. Control information may be received from an external source.

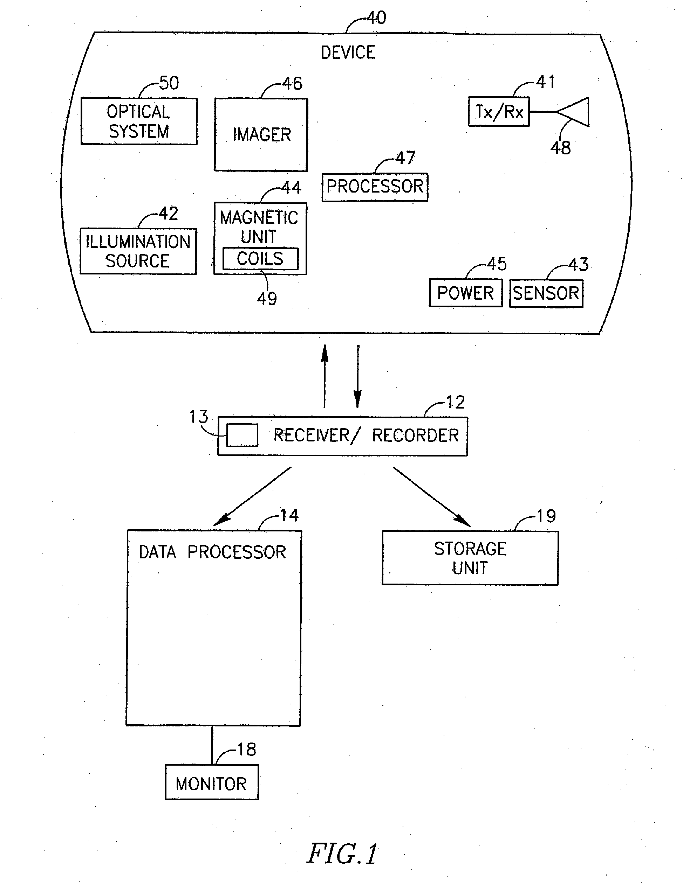

Figure 1 schematically illustrates an in-vivo system in accordance with some embodiments of the present invention. One or more components of the system may be used in conjunction with, or may be operatively associated with, the devices and/or components described herein or other in-vivo devices in accordance with embodiments of the invention.

The system includes a device 40 having an imager 46, one or more illumination sources 42, a power source 45, a magnetic unit 44 and a transmitter/receiver 41. In some embodiments, device 40 may be implemented using a swallowable capsule, but other sorts of devices or suitable implementations may be used. Outside a patient's body may be, for example, an external receiver/recorder 12 (including, or operatively associated with, for example, one or more antennas, or an antenna array), a storage unit 19, a processor 14, and a monitor 18. In some embodiments, for example, processor 14, storage unit 19 and/or monitor 18 may be implemented as a workstation, e.g., a computer or a computing platform.

Transmitter/receiver 41 may operate using radio waves; but in some embodiments, such as those where device 40 is or is included within an endoscope, transmitter/receiver 41 may transmit/receive data via, for example, wire, optical fiber and/or other suitable methods. Other known wireless methods of transmission may be used. Transmitter/receiver 41 may include, for example, a transmitter module or sub-unit and a receiver module or sub-unit, or an integrated transceiver or transmitter-receiver.

Device 40 typically may be or may include an autonomous swallowable capsule, but device 40 may have other shapes and need not be swallowable or autonomous. Embodiments of device 40 are typically autonomous, and are typically self-contained. For example, device 40 may be a capsule or other unit where all the components are substantially contained within a container or shell, and where device 40 does not require any wires or cables to, for example, receive power or transmit information. In some embodiments, device 40 may be autonomous and non-remote-controllable; in another embodiment, device 40 may be partially or entirely remote-controllable through a communication channel from the external receiver/recorder 12 to the device 40.

Device 40 may communicate with an external receiving and display system (e.g., monitor 18) to provide display of data, control, location or other functions. For example, power may be provided to device 40 using an internal battery, an internal power source, or a wireless system able to receive power. Other embodiments may have other configurations and capabilities. For example, components may be distributed over multiple sites or units, and control information or other information may be received from an external source.

Device 40 includes magnetic unit 44 which may include a magnetic field generator, for example, a conductive coil 49, a plurality of conductive coils 49, a permanent magnet, a plurality of permanent magnets, or any other magnetic field generator. The magnetic field unit may include a conductive coil, a current source or a power source that may be operatively connected to the conductive coil and a control unit which may be a switch or a controller to control the magnetic field generator. The power source may be included in magnetic unit 44, power source 45 or an external power source. In some embodiments magnetic unit 44 may include three mutually perpendicular coils, each may generate a magnetic field at a different time while receiver 12 may include at least three receiving antennas, for example, three mutually perpendicular coils. Based on the difference in the power received in each receiving antenna the position of the transmitting antennas may be determined. In some embodiments one coil may be included in magnetic unit 44 and three or more receiving antennas may be located outside the body in order to acquire a 3 dimensional position. Coils located outside the body are used as the generating magnetic field antennas while coils located in magnetic unit 44 are used as the receiving antennas.

The magnetic unit may be used as a voltage source for other units or elements in device 40, for example, a conductive coil and a voltage source may be used as power source for illumination source 42, optical system 50 or any other unit or electrical circuit in device 40. During the operation of magnetic unit 44 an AC current flows through the conductive coil. The AC current may be low frequency, for example in a range of 0.5-5 Mhz. The AC current may generate a magnetic field which may be used for determining the position of device 40. Other frequencies may be used. In some embodiments, voltage or current may be applied to one or more coils, for example, selectively, at pre-defined time intervals, substantially continuously, at one or more pre-defined time(s), on demand, in response to a remote instruction, in response to an external instruction, or the like. In some embodiments, voltage or current may be applied to one or more coils to generate magnetic fields simultaneously but with different frequencies. The pre-defined times may be, for example, after each transmitted video frame.

The magnetic field generated by magnetic unit 44 may be detected outside device 40, for example, outside the human body and may be used for position determination. For example a plurality of magnetic detectors, for example, magnetic sensors, coils, antennas or any other suitable detector may detect the magnetic field intensity at certain positions and orientations based on the detector locations. The detectors may be included in an external image receiving system, For example, external receiver/recorder 12 may be connected to a plurality of magnetic detectors or other suitable detectors which may be positioned at known locations and may detect, measure and record the magnetic field intensities detected by the magnetic detectors.

In some embodiments, magnetic unit 44 may include coil(s) which may receive a voltage of, for example, between 5 Volts and 15 Volts; other suitable voltage values may be used. In some embodiments, the radiation of magnetic unit 44 coil(s) may be at, for example, a frequency of between 0.5 MHz to 5 MHz; other suitable frequency values may be used.

Magnetic unit 44 is used as a receiving antenna, for example, a conductive coil, which may be the coil of a DC-DC converter, may be used for detecting magnetic field intensity. The coil of the DC-DC converter may function both as a receiving antenna and as an RF transmitting antenna. In some embodiments, the device 40 may include separate antennas for receiving and transmitting and a separate coil for the DC-DC converter. Other combinations are possible. For example, there may be a separate antenna for transmission and the coil of the DC-DC converter may function also as a receiving antenna. Or, there may be a separate antenna for receiving and the coil of the DC-DC converter may function also as a transmitting antenna. In some embodiments magnetic unit 44 may include one or more conductive coil(s) which may alternate between detecting magnetic field and supplying voltage to electrical circuits or units in device 40. The conductive coil or coils detect magnetic fields which may be generated outside of the human body by a plurality of magnetic field generators, for example, coils, magnets or other ferromagnetic materials. The magnetic field generators may be located on the body surface at known positions and may generate, create or transmit magnetic fields in a known order, such that the order of the detected magnetic field intensities may be known. In accordance with some embodiments, the magnetic unit 44 may include three mutually perpendicular coils and position determination may be based on the difference in power received in each of the coils by the magnetic fields generated outside the body.

In some embodiments magnetic unit 44 may include one or more coils, for example, to generate magnetic fields and one or more coils to detect magnetic fields. In some embodiments all the coils included in magnetic unit 44 may be used to generate magnetic fields, in other embodiments all the coils included in magnetic unit 44 may be used as magnetic field detectors. In some embodiments some of the coils included in magnetic unit 44 may be used to generate magnetic fields while some of the coils included in magnetic unit 44 may be used to detect magnetic fields. In some embodiments the same coils included in magnetic unit 44 may be used for both generating and detecting magnetic fields.

Magnetic unit 44 may be located, for example, near or at external portion(s) of device 40, e.g., close to the exterior of device 40, embedded within a housing (e.g., a capsule housing) which may enclose or encapsulate the components of device 40, or the like. In some embodiments, magnetic unit 44 may be external to device 40.

Device 40 includes an imager 46, which may capture and transmit images of, for example, the GI tract while device 40 passes through the GI lumen. Other lumens and/or body cavities may be imaged and/or sensed by device 40. In some embodiments, imager 46 may include, for example, a Charge Coupled Device (CCD) camera or imager, a Complementary Metal Oxide Semiconductor (CMOS) camera or imager, a digital camera, a stills camera, a video camera, or other suitable imagers, cameras, or image acquisition components.

Imager 46 in device 40 is operationally connected to transmitter/receiver 41. Transmitter/receiver 41 transmits images to, for example, external transceiver or receiver/recorder 12 (through one or more antennas 48), which may send the data to processor 14 and/or to storage unit 19. Transmitter/receiver 41 may also include control capability, although control capability may be included in a separate component, e.g., processor 47. Transmitter/receiver 41 may include any suitable transmitter able to transmit image data, other sensed data, and/or other data (e.g., control data) to a receiving device. Transmitter/receiver 41 may also be capable of receiving signals/commands, for example from an external transceiver. For example, in some embodiments, transmitter/receiver 41 may include an ultra low power Radio Frequency (RF) high bandwidth transmitter, possibly provided in Chip Scale Package (CSP).

Transmitter/receiver 41 transmits receives via antenna 48. Transmitter/receiver 41 and/or another unit in device 40, e.g., a controller or processor 47, may include control capability, for example, one or more control modules, processing module, circuitry and/or functionality for controlling device 40, for controlling the operational mode or settings of device 40, and/or for performing control operations or processing operations within device 40. According to some embodiments, transmitter/receiver 41 may include a receiver which may receive signals (e.g., from outside the patient's body), for example, through antenna 48 or through a different antenna or receiving element. According to some embodiments, signals or data may be received by a separate receiving device in device 40.

Power source 45 may include one or more batteries or power cells. For example, power source 45 may include silver oxide batteries, lithium batteries, other suitable electrochemical cells having a high energy density, or the like. Other suitable power sources may be used. For example, power source 45 may receive power or energy from an external power source (e.g., an electromagnetic field generator), which may be used to transmit power or energy to in-vivo device 40.

In some embodiments, power source 45 may be internal to device 40, and/or may not require coupling to an external power source, e.g., to receive power. Power source 45 may provide power to one or more components of device 40 continuously, substantially continuously, or in a non-discrete manner or timing, or in a periodic manner, an intermittent manner, or an otherwise non-continuous manner. In some embodiments, power source 45 may provide power to one or more components of device 40, for example, not necessarily upon-demand, or not necessarily upon a triggering event or an external activation or external excitement.

Optionally, in some embodiments, transmitter/receiver 41 may include a processing unit or processor or controller, for example, to process signals and/or data generated by imager 46. In another embodiment, the processing unit may be implemented using a separate component within device 40, e.g., controller or processor 47, or may be implemented as an integral part of imager 46, transmitter/receiver 41, or another component, or may not be needed. The processing unit may include, for example, a Central Processing Unit (CPU), a Digital Signal Processor (DSP), a microprocessor, a controller, a chip, a microchip, a controller, circuitry, an Integrated Circuit (IC), an Application-Specific Integrated Circuit (ASIC), a Field Programmable Gate Array (FPGA), or any other suitable multi-purpose or specific processor, controller, circuitry or circuit. In some embodiments, for example, the processing unit or controller may be embedded in or integrated with transmitter/receiver 41, and may be implemented, for example, using an ASIC.

In some embodiments, imager 46 may acquire in-vivo images continuously, substantially continuously, or in a non-discrete manner, for example, not necessarily upon-demand, or not necessarily upon a triggering event or an external activation or external excitement; or in a periodic manner, an intermittent manner, or an otherwise non-continuous manner.

In some embodiments, transmitter/receiver 41 may transmit image data continuously, or substantially continuously, for example, not necessarily upon-demand, or not necessarily upon a triggering event or an external activation or external excitement; or in a periodic manner, an intermittent manner, or an otherwise non-continuous manner.

In some embodiments, device 40 may include one or more illumination sources 42, for example one or more Light Emitting Diodes (LEDs), "white LEDs", or other suitable light sources. Illumination sources 42 may, for example, illuminate a body lumen or cavity being imaged and/or sensed. An optional optical system 50, including, for example, one or more optical elements, such as one or more lenses or composite lens assemblies, one or more suitable optical filters, or any other suitable optical elements, may optionally be included in device 40 and may aid in focusing reflected light onto image 46, focusing illuminated light, and/or performing other light processing operations.

In some embodiments, illumination source(s) 42 may illuminate continuously, or substantially continuously, for example, not necessarily upon-demand, or not necessarily upon a triggering event or an external activation or external excitement. In some embodiments, for example, illumination source(s) 42 may illuminate a pre-defined number of times per second (e.g., two or four times), substantially continuously, e.g., for a time period of two hours, four hours, eight hours, or the like; or in a periodic manner, an intermittent manner, or an otherwise non-continuous manner. In some embodiments illumination source(s) 42 may be operatively connected to a conductive coil, e.g., magnetic unit 44, and a power source, e.g., power source 45. The coil may be the coil of a DC-DC converter for providing high DC voltage to the illumination source(s) 42.

In some embodiments, the components of device 40 may be enclosed within a housing or shell, e.g., capsule-shaped, oval, or having other suitable shapes. The housing or shell may be substantially transparent or semi-transparent, and/or may include one or more portions, windows or domes which may be substantially transparent or semi-transparent. For example, one or more illumination source(s) 42 within device 40 may illuminate a body lumen through a transparent or semi-transparent portion, window or dome; and light reflected from the body lumen may enter the device 40, for example, through the same transparent or semi-transparent portion, window or dome, or, optionally, through another transparent or semi-transparent portion, window or dome, and may be received by optical system 50 and/or imager 46. In some embodiments, for example, optical system 50 and/or imager 46 may receive light, reflected from a body lumen, through the same window or dome through which illumination source(s) 42 illuminate the body lumen.

Data processor 14 may analyze the data received via external receiver/recorder 12 from device 40, and may be in communication with storage unit 19, e.g., transferring frame data to and from storage unit 19. Data processor 14 may provide the analyzed data to monitor 18, where a user (e.g., a physician) may view or otherwise use the data. In some embodiments, data processor 14 may be configured for real time processing and/or for post processing to be performed and/or viewed at a later time. In the case that control capability (e.g., delay, timing, etc) is external to device 40, a suitable external device (such as, for example, data processor 14 or external receiver/recorder 12 having a transmitter or transceiver) may transmit one or more control signals to device 40.

Monitor 18 may include, for example, one or more screens, monitors, or suitable display units. Monitor 18, for example, may display one or more images or a stream of images captured and/or transmitted by device 40, e.g., images of the GI tract or of other imaged body lumen or cavity. Additionally or alternatively, monitor 18 may display, for example, control data, location or position data (e.g., data describing or indicating the location or the relative location of device 40), orientation data, and various other suitable data. In some embodiments, for example, both an image and its position (e.g., relative to the body lumen being imaged) or location may be presented using monitor 18 and/or may be stored using storage unit 19. Other systems and methods of storing and/or displaying collected image data and/or other data may be used.

Typically, device 40 can transmit image information in discrete portions. Each portion may typically correspond to an image or a frame.

Typically, the image data recorded and transmitted may include digital color image data; in alternate embodiments, other image formats (e.g., black and white image data) may be used. In some embodiments, each frame of image data may include 256 rows, each row may include 256 pixels, and each pixel may include data for color and brightness according to known methods. According to other embodiments a 320x320 pixel imager may be used. Pixel size may be between 5 to 6 micron. According to some embodiments pixels may be each fitted with a micro lens. For example, a Bayer color filter may be applied. Other suitable data formats may be used, and other suitable numbers or types of rows, columns, arrays, pixels, sub-pixels, boxes, super-pixels and/or colors may be used.

Optionally, device 40 may include one or more sensors 43, instead of or in addition to a sensor such as imager 46. Sensor 43 may, for example, sense, detect, determine and/or measure one or more values of properties or characteristics of the surrounding of device 40. For example, sensor 43 may include a pH sensor, a temperature sensor, an electrical conductivity sensor, a pressure sensor, or any other known suitable in-vivo sensor.

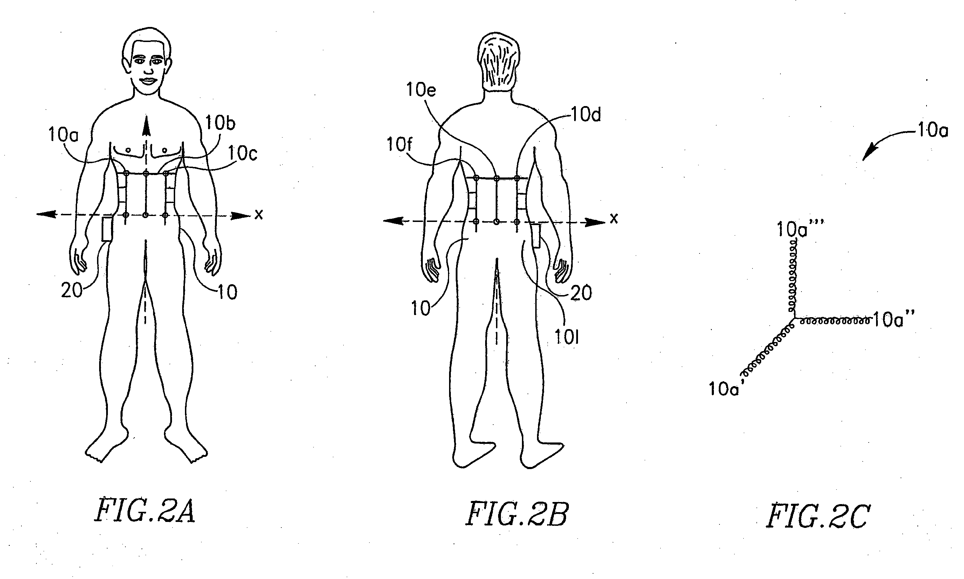

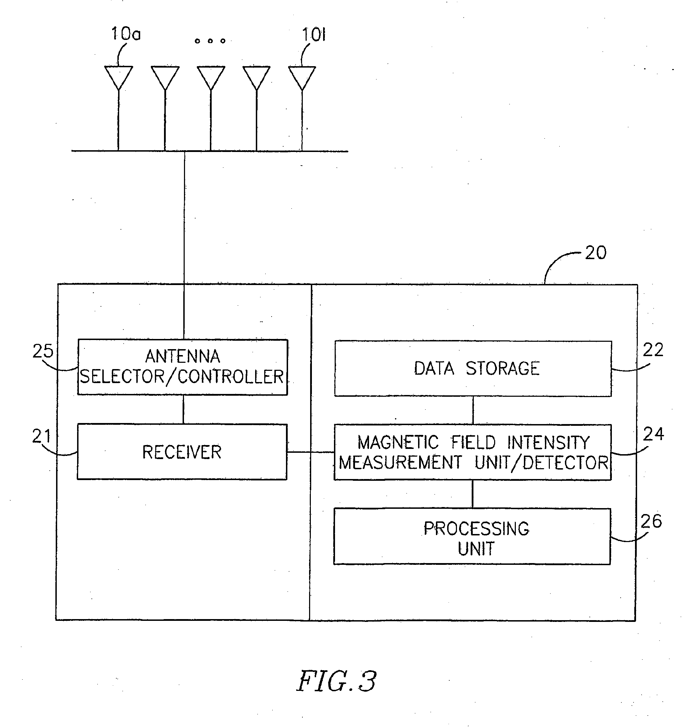

Figs. 2A and 2B schematically illustrate a patient wearing a magnetic antenna array according to an embodiment of the present invention. According to an embodiment of the present invention, the position of an in-vivo magnetic field source, for example, an in-vivo device 40 may be detected, located or localized using a portable or wearable magnetic antenna array or magnetic antenna array belt 10, as shown in Figs. 2A and 2B. In other embodiments the magnetic antenna array may be integral to a jacket that the patient may wear. The magnetic antenna array belt 10 may be fitted such that it may be wrapped around a patient and attached to a signal recorder 20 (see Fig. 3). Additional embodiments include, for example, magnetic antenna elements having adhesive, which may adhere the element to a point on a body. Magnetic antenna array 10 contains one or more magnetic antenna elements (10a, 10b, ...) which may be, for example, magnetic sensors, conductive coils, magnetic detectors or other elements which are used for detection and generation of magnetic fields. In accordance with some embodiments, magnetic antenna array 10 may consist of a single magnetic antenna element 10a comprising a set of three magnetic antennas. In accordance with some embodiments, the three magnetic antennas may be three mutually perpendicular coils 10a', 10a", 10a'", as shown in Fig. 2C. The antenna element 10a may be connected via a coaxial cable to a connector, which may connected to the recorder 20. In some embodiments the number of magnetic antenna elements may be more than one, with each antenna element being a multiple of three magnetic antennas. Each magnetic antenna may be a magnetic field detector, a magnetic sensor, a conductive coil or another suitable magnetic field detector or generator configuration. for example, in one embodiment nine antenna elements 10a through 10i may be used.

In one embodiment, the antenna array belt 10 may include twelve antenna elements, for example, antenna elements 10a through 10L, which may be typically positioned on a patient's midsection. Each of the twelve antenna elements 10a through 10L may comprise three perpendicular coils.

Each magnetic antenna is used for generating a magnetic field and for detecting a magnetic field. The magnetic field may be generated by, for example, magnetic unit 44 of in vivo device 40. Each measurement detected by magnetic antennas 10a to 10L may be transferred to signal recorder 20 and may be used for position location of in vivo device 40. In one embodiment each one of magnetic antennas 10a to 10L may generate a magnetic field in a known order, for example; each of the magnetic antennas 10a to 10L may generate a magnetic field in a predetermined known order for a predetermined period of time. For example, each antenna may generate a magnetic field for 20 seconds one after the other. In accordance with some embodiments, each antenna may generate a magnetic field simultaneously but at different frequencies. Magnetic field generation may be controller by a control unit 13 which may be located in, for example, signal recorder 20, receiver 12 or by a separate control unit. The order of the magnetic field generation may be changed, programmed before and/or during operation of device 40. For example, after each transmitted video frame. The magnetic fields generated by magnetic antenna array 10 are detected by magnetic unit 44 of device 40. The magnetic field intensities detected by magnetic unit 44, are transmitted outside of the body, using RF waves, by means of a telemetry channel along with image data transmitted by device 40. The transmitted measurements of the magnetic field intensities may be recorded by signal recorder 12 and may be processed to obtain the position location of device 40. The position determination may incorporate information of the near field and far field patterns of the magnetic antenna array 10 The position determination is performed by processing unit 26. Other position determination methods may be performed as may have been described herein and are in accordance with embodiments of the invention.

Fig. 3 schematically illustrates a data recorder 20 according to an embodiment of the present invention. Data recorder 20 may include, for example, a data storage unit 22, a receiver 21, a magnetic field intensity measurement unit and/or a magnetic field intensity detector 24, a processing unit 26 and an antenna selector and/or antenna controller 25. In alternate embodiments, the data recorder 20 may include other combinations of components, and the components described may be divided among several or other units.

In some embodiments of the present invention, the antenna array 10 may include a plurality of antennas wherein the antennas may detect magnetic fields from a plurality of locations, for example magnetic fields generated by an in-vivo magnetic generator. In some embodiments of the present invention, antenna array 10 may include a plurality of antennas wherein the antennas may generate a magnetic field from a plurality of locations in a predetermined known order. In some embodiments of the present invention, the antenna array may include a plurality of antennas wherein the antennas may receive a signal and/or information from a plurality of locations, for example by an RF signal, transmitted from the in-vivo image sensor. For example in some embodiments wherein the antennas may generate a magnetic field from a plurality of locations in a known order and the in vivo device, for example, device 40 detects and measures the magnetic fields. Measured magnetic field intensity values may be transmitted in the telemetry channel by, for example, an RF signal which may contain additional information such as image data. The transmitted signal may be received by receiver 21 or receiver 12 of Fig. 1. In some embodiments antenna selector 25 may open a signal path to one or more antenna elements from which the receiver 21 will receive a signal, e.g., a magnetic field intensity measurement. Antenna controller 25 controls the order in which the antenna elements generate a magnetic field and the order and duration of generating the magnetic fields is at pre-defined times, after each transmitted video frame.

The magnetic field intensity measurement unit 24 may measure the magnetic field intensities of signals received by receiver 21 from a plurality of locations, for example, from each of antenna elements 10a through 10L.

Antenna selector 25 may be adjusted to scan through all or subset of antenna elements 10a through 10L. The scan rate and order of scanning may be adjusted.

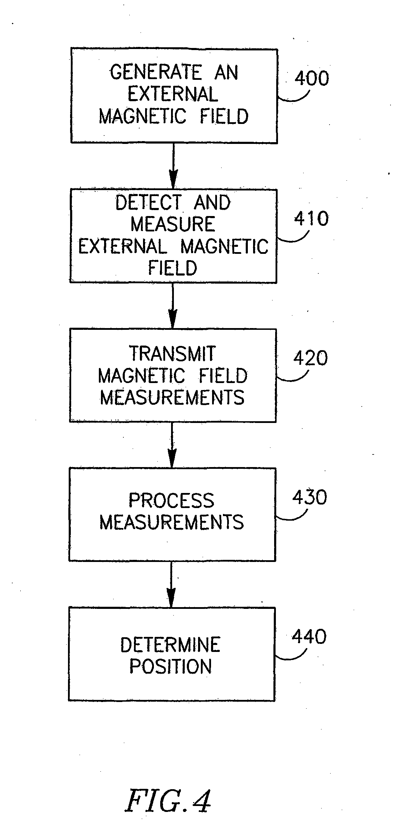

Figure 4 is a schematic flow-chart of a method of in-vivo magnetic position detection in accordance with the invention. The method is used in conjunction with systems described herein.

In block 400, external electro-magnetic fields are generated by magnetic field generators located in antenna array 10 of Fig. 2. The external magnetic fields are generated in a predetermined, known order that is controled by antenna controller 25 of Fig. 3. The order of the magnetic fields generation and application may be, for example, all magnetic field generators generating magnetic fields in a predetermined order one after the other for a predetermined period of time, a reduced set of the magnetic generators generating magnetic fields in a predetermined order one at a time for a predetermined period of time or any other order. In accordance with some embodiments, the magnetic fields may be generated simultaneously but at different frequencies.

In block 410, a receiving antenna, for example, a conductive coil, a magnetic sensor or other suitable magnetic detector may detect, sense, and measure the external electro-magnetic fields applied in block 400. The receiving antenna is located in unit 44 of device 40 of Fig. 1. The receiving antenna may be used for other functions during time periods in which magnetic fields are not to be detected, for example, if the antenna is a conductive coil it may be used as a part of other electronic circuits such as a DC-DC converter, or it may be used for receiving RF transmissions and/or transmitting signals. Any other use or operations may be performed.

In block 420, the measured magnetic fields results, e.g., measured magnetic field intensities of the detected magnetic fields are transmitted to receiver 21 of Fig. 3. The magnetic field measurements are transmitted from device 40 of Fig. 1 to receiver 21 of Fig. 3. The transmission of magnetic field measurements is performed from antenna 48 of Fig. 1.

In block 430, the transmitted magnetic field measurements are processed by processing unit 26 of Fig. 3. The processing unit may be an internal processing unit or external processing unit In some embodiments the processing of the magnetic field measurements may be performed while the detection of the magnetic fields is in process, e,g, real time processing, while in other embodiments the processing of the magnetic field measurements may be performed after detection of the magnetic fields, e.g., using off line processing.

In block 440, determination of the position of the device 40 is performed. For example the position detection may include calculations of distances, for example, by correlating the detected magnetic field measurements with an estimated location of the source of the signal. In accordance with some embodiments, the magnetic unit 44 may include three mutually perpendicular coils and position determination may be based on the difference in power received in each of the coils. The position detection is performed by processing unit 26 of Fig. 3. The position detection may also be performed off-line. Other position detection methods may be performed as may have been described herein and may be in accordance with embodiments of the invention.

It is noted that some or all of the above-mentioned operations may be performed substantially in real time, e.g., during the operation of the in-vivo imaging device, during the time in which the in-vivo imaging device operates and/or captures images, and/or without interruption to the operation of the in-vivo imaging device. Other operations or sets of operations may be used in accordance with embodiments of the invention.

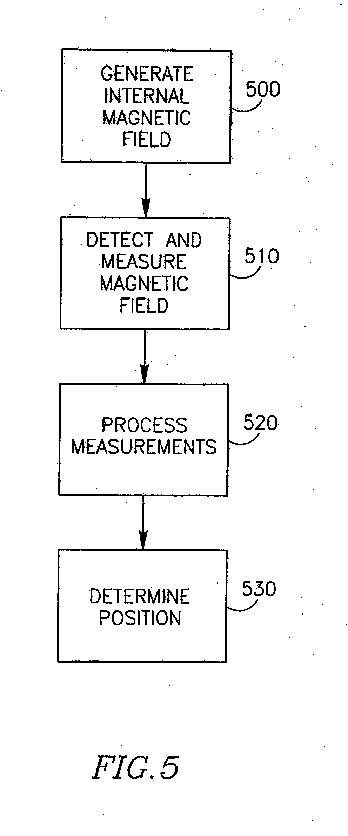

Figure 5 is a schematic flow-chart of a method of in-vivo magnetic position detection in accordance with some embodiments of the invention. The method may be used, for example, in conjunction with one or more components, devices and/or systems described herein, and/or other suitable in-vivo devices and/or systems.

In block 500, an internal magnetic field may be generated by, for example, magnetic generator located in magnetic unit 44 of Fig. 1. The magnetic generator may be for example, a conductive coil which may be used as a voltage source to other units or circuits, for example, may be used as a power source to illumination source 42 or optical system 50 of Fig. 1. In some embodiments, during the operation of the magnetic generator an AC current flows through the conductive coil, the AC current may be low frequency, for example in a range of 0.5-5 MHz, this AC current may generate a magnetic field. The conductive coil may be, for example, the coil of a DC-DC converter,

In block 510, receiving antennas, for example, antenna array 10 of Fig. 2 may detect, sense, and measure the received internal electro-magnetic field. For example, the magnetic field intensity may be measured. The detection and measurement of the magnetic field may be performed by plurality of antennas located at known positions, for example, the antennas array may be fitted such that it may be wrapped around a patient and attached to a belt The receiving antennas may contain one or more magnetic antenna elements which may be, for example, magnetic sensors, conductive coils, magnetic detectors or other elements which may be used for detection and/or generation of magnetic field. For example, magnetic antenna array 10 may contain a set of at least three magnetic antennas which may be three mutually perpendicular coils.

In block 520, the transmitted magnetic field measurements may be processed by a processing unit, for example, processing unit 26 of Fig. 3, data processor 14 of Fig. 1 or processor 47 of Fig. 1. The processing unit may be an internal processing unit or external processing unit. In some embodiments the processing of the magnetic field measurements may be performed while the detection of the magnetic fields is in process, e,g, real time processing, while in other embodiments the processing of the magnetic field measurements may be performed after detection of the magnetic fields, e.g., using off line processing.

In block 530, a determination of the position of the in vivo device, for example, device 40 of Fig. 1 may be performed. The magnetic field measurements may be processed by a processing unit, for example, processing unit 26 of Fig. 3, data processor 14 of Fig. 1 or processor 47 of Fig. 1. In accordance with some embodiments, the detection and measurement of the magnetic field may be performed by magnetic antenna array 10 comprising at least one antenna element 10a. The antenna element 10a may include three mutually perpendicular coils and position determination may be based on the difference in power received in each of the coils. The position determination may be performed by an internal processing unit or external processing unit. In some embodiments the position determination may be performed while the detection of the magnetic fields is on process, e,g, real time processing, while in other embodiments the position determination may be performed after detection of the magnetic fields, e.g., using off line processing. Other position determination methods may be performed as may have been described herein and may be in accordance with embodiments of the invention.

The determined position may be for example relative to receiving units such as antennas 10, relative to for example a patient's body or features of a patient's body, relative to previous positions of the in-vivo device 40, or expressed in different terms. For example, the position may be relative to an outline of the patient's torso, or to a representation of the GI tract, or the position may be used to generate a map of the path of the device, where the position calculated is relative to a set of positions of the device, for example, device 40 as is passes through the GI tract

It is noted that some or all of the above-mentioned operations may be performed substantially in real time, e.g., during the operation of the in-vivo imaging device, during the time in which the in-vivo imaging device operates and/or captures images, and/or without interruption to the operation of the in-vivo imaging device. Other operations or sets of operations may be used in accordance with embodiments of the invention.

Various aspects of the various embodiments disclosed herein are combinable with the other embodiments disclosed herein.

A device, system and method in accordance with some embodiments of the invention may be used, for example, in conjunction with a device which may be inserted into a human body. However, the scope of the present invention is not limited in this regard. For example, some embodiments of the invention may be used in conjunction with a device which may be inserted into a non-human body or an animal body.

## Claims

1. A system for determining the position of an in-vivo sensing device comprising:
- an in vivo sensing device (40) comprising:
- an imager (46) for capturing images in vivo,
- an illumination source (42),
- a power source (45),
- an antenna (48),
- a transmitter/receiver (41) to transmit captured images via the antenna (48) in discrete portions, wherein each discrete portion corresponds to a video frame, and
- a magnetic unit (44);
- a signal recorder (20) comprising an antenna controller (25), a data storage unit (22), a receiver (21) and a processing unit (26);
- a magnetic antenna array (10) comprising at least one magnetic antenna element (10a, 10b, ...), wherein each magnetic antenna element (10a, 10b, ...) is suitable for generating a magnetic field, wherein a single magnetic antenna element (10a, 10b, ...) comprises at least one set of three mutually perpendicular coils (10a', 10a", 10a"'), wherein the at least one magnetic antenna element (10a, 10b, ...) is portable or wearable by a patient;
wherein:
- the antenna array (10) is attached to the antenna controller (25), wherein the antenna controller (25) is adapted to control the order in which the antenna elements (10a, 10b, ...) generate a magnetic field and the order and duration of generating the magnetic fields is at predetermined times and after each transmitted video frame;
- the antenna controller (25) is configured to, after each transmission of a video frame by the transmitter (41), generate the magnetic field by the antenna elements (10a) of the antenna array (10),
- the magnetic unit (44) is configured to, after each transmission of a video frame by the transmitter (41), detect the magnetic field of the antenna elements (10a) of the antenna array (10) and measure magnetic field values of the detected magnetic fields,
- the transmitter (41) is configured to transmit the measured magnetic field values from the antenna (48) to the receiver (21), and
- the processing unit (26) is configured to determine the position of the in-vivo sensing device (40) from the transmitted measured magnetic field values.

2. The system according to claim 1, wherein voltage is applied to the magnetic unit (44) in response to an external instruction.

3. The system according to claim 1, wherein the magnetic unit (44) includes a power source which is used as a voltage source for elements in the in vivo sensing device (40).

4. The system according to any of claims 1 to 3, wherein the magnetic unit (44) comprises one or more conductive coils (49), and wherein the one or more conductive coils (49) are configured to be alternately used to detect magnetic fields and to supply voltage to units in the in-vivo device (40).

5. The system according to any of claims 1 to 4, wherein the magnetic unit (44) comprises at least one set of three mutually perpendicular coils (49).

6. The system according to any of claims 1 to 5, wherein the magnetic unit (44) comprises one or more conductive coils (49).

7. The system according to claim 6, wherein the magnetic unit (44) comprises three mutually perpendicular coils (49).

8. A method of using the system according to claims 1 to 7 for determining the position of an in-vivo sensing device (40) comprising an imager (46), which is an in-vivo video camera, said method comprising the steps of:
- capturing images in vivo by the imager (46);
- transmitting captured images via an antenna (48) included in the in-vivo sensing device (40) in discrete portions, wherein each discrete portion corresponds to a video frame;
- after each video frame transmission, performing the steps of:
(i) generating magnetic fields (400) by a magnetic field generator which is external to the in-vivo sensing device (40); and
(ii) detecting the magnetic fields by a magnetic unit (44) included in the in-vivo sensing device (40), and measuring magnetic field values of the detected magnetic fields;
- transmitting the measured magnetic field values from the antenna (48) to an external unit; and
- determining the position of the in-vivo sensing device (40) from the transmitted magnetic field values.

9. The method according to claim 8, wherein the magnetic fields are generated by at least one set of three mutually perpendicular coils (10a', 10a", 10a"').

10. The method according to any of claims 8 or 9, wherein the magnetic unit (44) includes a power source which is used as a voltage source for elements in the in vivo sensing device (40).

11. The method according to claim 10, wherein the position of the in-vivo device is determined from a power induced in each coil by the magnetic fields.

12. The method according to claim 9 or 10, comprising alternating the one or more conductive coils (49) between detecting magnetic fields and supplying voltage to units in the in-vivo sensing device (40).

13. The method according to any of claims 8 to 12, wherein the magnetic fields generated by the magnetic field generator are generated at different times.

14. The method according to any of claims 8 to 13, wherein the magnetic fields generated by the magnetic field generator are generated simultaneously with different frequencies.

## Patentansprüche

1. System zum Bestimmen der Position einer In-vivo-Sensorvorrichtung, umfassend:
- eine In-vivo-Sensorvorrichtung (40), umfassend:
- einen Bildgeber (46) zur Aufnahme von Bildern in vivo,
- eine Lichtquelle (42),
- eine Energiequelle (45),
- eine Antenne (48),
- einen Sender/ Empfänger (41) zum Übertragen von aufgenommenen Bildern in diskreten Teilen über die Antenne (48), wobei jeder diskrete Teil einem Video-Frame entspricht, und
- eine Magneteinheit (44);
- einen Signalrecorder (20) umfassend eine Antennensteuereinheit (25), einen Datenspeicher (22), einen Empfänger (21) und eine Verarbeitungseinheit (26);
- eine magnetische Antennenanordnung (10) umfassend zumindest ein magnetisches Antennenelement (10a, 10b,...), wobei jedes magnetische Antennenelement (10a, 10b,...) geeignet ist zum Erzeugen eines Magnetfeldes, wobei ein einzelnes magnetisches Antennenelement (10a, 10b,...) zumindest einen Satz von drei zueinander senkrechten Spulen (10a', 10a", 10a'") umfasst, wobei das zumindest eine magnetische Antennenelement (10a, 10b,...) beweglich oder tragbar ist durch einen Patienten;
wobei:
- die Antennenanordnung (10) an der Antennensteuereinheit (25) befestigt ist, wobei die Antennensteuereinheit (25) ausgestaltet ist die Reihenfolge zu steuern, in welcher die Antennenelemente (10a, 10b,...) ein Magnetfeld erzeugen, wobei die Reihenfolge und Dauer der Erzeugung der Magnetfelder zu vorherbestimmten Zeiten und nach jedem übertragenen Video-Frame erfolgt;
- die Antennensteuereinheit (25) ist ausgestaltet um nach jeder Übertragung eines Video-Frames durch den Sender (41) das magnetische Feld durch die Antennenelemente (10a) des Antennen-Arrays (10) zu erzeugen,
- die Magneteinheit (44) ausgestaltet ist, um nach jeder Übertragung eines Video-Frame durch der Sender (41) das Magnetfeld der Antennenelemente (10a) des Antennen-Arrays (10) zu erkennen und die Magnetfeldwerte der erkannten Magnetfelder zu messen,
- der Sender (41) konfiguriert ist, um die gemessenen Magnetfeldwerte von der Antenne (48) zum Empfänger (21) zu übertragen, und
- die Verarbeitungseinheit (26) konfiguriert ist, um die Position der In-vivo-Sensorvorrichtung (40) aus den übertragenen gemessenen Magnetfeldwerten zu bestimmen.

2. System nach Anspruch 1, wobei als Reaktion auf einen externen Befehl Spannung an die Magneteinheit (44) angelegt wird.

3. Das System nach Anspruch 1, wobei die Magneteinheit (44) eine Energiequelle umfasst, die als eine Spannungsquelle für die Elemente in der In-vivo-Sensoreinrichtung (40) verwendet wird.

4. Das System nach einem der Ansprüche 1 bis 3, wobei die Magneteinheit (44) eine oder mehrere leitende Spulen (49) umfasst, und wobei die eine oder mehrere leitende Spulen (49) konfiguriert sind, um abwechselnd verwendet zu werden, um Magnetfelder zu erfassen und um Einheiten in der In-vivo-Vorrichtung (40) mit Spannung zu versorgen.

5. System nach einem der Ansprüche 1 bis 4, wobei die Magneteinheit (44) mindestens einen Satz von drei zueinander senkrechten Spulen (49) umfasst.

6. System nach einem der Ansprüche 1 bis 5, wobei die Magneteinheit (44) eine oder mehrere leitende Spulen (49) umfasst.

7. System nach Anspruch 6, wobei die Magneteinheit (44) drei zueinander senkrechte Spulen (49) umfasst.

8. Verfahren zur Verwendung des Systems nach einem er Ansprüche 1 bis 7 zum Bestimmen der Position einer In-vivo-Sensorvorrichtung (40) umfassend einen Bildgeber (46), welcher eine In-vivo-Videokamera ist, wobei das Verfahren die folgenden Schritte umfasst:
- Aufnehmen von Bildern in vivo durch den Bildgeber (46);
- Übertragen der aufgenommenen Bilder in diskreten Teile über eine Antenne (48), wobei die Antenne (48) in der In-vivo-Sensorvorrichtung (40) enthalten ist, wobei jeder diskrete Teil einem Video-Frame entspricht;
- nach jeder Video-Frame-Übertragung, Ausführen der folgenden Schritte:
(i) Erzeugen von Magnetfeldern (400) durch einen Magnetfeldgenerator, der ausserhalb der In-vivo-Sensorvorrichtung (40) ist; und
(ii) Detektieren der Magnetfelder durch eine Magneteinheit (44), welche in der In-vivo-Sensorvorrichtung (40) enthalten ist, und Messen von Magnetfeldwerten der detektierten Magnetfeldern;
- Übertragen der gemessenen Magnetfeldwerten von der Antenne (48) zu einer externen Einheit; und
- Bestimmen der Position der In-vivo-Sensorvorrichtung (40) aus den übertragenen Magnetfeldwerten.

9. Verfahren nach Anspruch 8, wobei die Magnetfelder durch zumindest einen Satz von drei zueinander senkrechten Spulen (10a', 10a", 10a"') erzeugt werden.

10. Verfahren nach einem der Ansprüche 8 oder 9, wobei die Magneteinheit (44) eine Energiequelle umfasst, die als eine Spannungsquelle für die Elemente in der In-vivo-Sensorvorrichtung (40) verwendet wird.

11. Verfahren nach Anspruch 10, wobei die Position der In-vivo-Vorrichtung aus einer Energie bestimmt wird, welcher durch die Magnetfelder in jeder Spule induziert wird.

12. Verfahren nach Anspruch 9 oder 10, umfassend ein Abwechseln der einen oder mehreren leitenden Spulen (49) zwischen dem Erfassen von Magnetfeldern und dem Zuführen von Spannung zu Einheiten in der In-vivo-Sensorvorrichtung (40).

13. Verfahren nach einem der Ansprüche 8 bis 12, wobei die durch den Magnetfeldgenerator erzeugten Magnetfelder zu unterschiedlichen Zeiten erzeugt werden.

14. Verfahren nach einem der Ansprüche 8 bis 13, wobei die durch den Magnetfeldgenerator erzeugten Magnetfelder gleichzeitig mit unterschiedlichen Frequenzen erzeugt werden.

## Revendications

1. Système destiné à déterminer la position d'un dispositif de détection in vivo comprenant :
- un dispositif de détection in vivo (40) comprenant :
- un dispositif de formation d'images (46) destiné à acquérir des images in vivo,
- une source d'illumination (42),
- une source d'alimentation (45),
- une antenne (48),
- un émetteur/récepteur (41) destiné à transmettre des images acquises via l'antenne (48) en parties discrètes, où chaque partie discrète correspond à une trame vidéo, et
- un enregistreur de signal (20) comprenant un contrôleur d'antenne (25), une unité de mémorisation de données (22), un récepteur (21) et une unité de traitement (26),
- un réseau d'antennes magnétiques (10) comprenant au moins un élément d'antenne magnétique (10a, 10b, ...), où chaque élément d'antenne magnétique (10a, 10b, ...) est approprié pour générer un champ magnétique, où un seul élément d'antenne magnétique (10a, 10b, ...) comprend au moins un ensemble de trois bobines mutuellement perpendiculaires (10a', 10a", 10a"'), où le au moins un élément d'antenne magnétique (10a, 10b, ...) est portatif ou portable par un patient,
où :
- le réseau d'antennes (10) est fixé au contrôleur d'antenne (25), où le contrôleur d'antenne (25) est conçu pour commander l'ordre dans lequel les éléments d'antenne (10a, 10b, ...) génèrent le champ magnétique et l'ordre et la durée de la génération du champ magnétique à des instants prédéterminés et après chaque trame vidéo transmise,
- le contrôleur d'antenne (25) est configuré pour, après chaque transmission d'une trame vidéo par l'émetteur (41), générer le champ magnétique avec les éléments d'antenne (10a) du réseau d'antennes (10),
- l'unité magnétique (44) est configurée pour, après chaque transmission d'une trame vidéo par l'émetteur (41), détecter le champ magnétique des éléments d'antenne (10a) du réseau d'antennes (10) et mesurer les valeurs de champs magnétiques des champs magnétiques détectés,
- l'émetteur (41) est configuré pour transmettre les valeurs de champs magnétiques mesurées de l'antenne (48) au récepteur (21), et
- l'unité de traitement (26) est configurée pour déterminer la position du dispositif de détection in vivo (40) à partir des valeurs de champs magnétiques mesurées transmises.

2. Système selon la revendication 1, dans lequel une tension est appliquée à l'unité magnétique (44) en réponse à une instruction externe.

3. Système selon la revendication 1, dans lequel l'unité magnétique (44) comprend une source d'alimentation qui est utilisée comme source de tension pour des éléments dans le dispositif de détection in vivo (40).

4. Système selon l'une quelconque des revendications 1 à 3, dans lequel l'unité magnétique (44) comprend une ou plusieurs bobines conductrices (49), et dans lequel les une ou plusieurs bobines conductrices (49) sont configurées pour être alternativement utilisées pour détecter des champs magnétiques et pour fournir une tension aux unités dans le dispositif de détection in vivo (40).

5. Système selon l'une quelconque des revendications 1 à 4, dans lequel l'unité magnétique (44) comprend au moins un ensemble de trois bobines mutuellement perpendiculaires (49).

6. Système selon l'une quelconque des revendications 1 à 5, dans lequel l'unité magnétique (44) comprend une ou plusieurs bobines conductrices (49).

7. Système selon la revendication 6, dans lequel l'unité magnétique (44) comprend trois bobines mutuellement perpendiculaires (49).

8. Procédé d'utilisation du système selon les revendications 1 à 7 destiné à déterminer la position d'un dispositif de détection in vivo (40) comprenant un dispositif de formation d'images (46), qui est une caméra vidéo in vivo, ledit procédé comprenant les étapes consistant à :
- acquérir des images in vivo avec le dispositif de formation d'images (46),
- transmettre les images acquises via une antenne (48) incluse dans le dispositif de détection in vivo (40) en parties discrètes, où chaque partie discrète correspond à une trame vidéo,
- après chaque transmission de trame vidéo, réaliser les étapes consistant à :
(i) générer des champs magnétiques (400) avec un générateur de champs magnétiques qui est externe au dispositif de détection in vivo (40), et
(ii) détecter les champs magnétiques avec une unité magnétique (44) incluse dans le dispositif de détection in vivo (40), et mesurer des valeurs de champs magnétiques des champs magnétiques détectés,
- transmettre des valeurs de champs magnétiques mesurées de l'antenne (48) vers une unité externe, et
- déterminer la position du dispositif de détection in vivo (40) à partir des valeurs de champs magnétiques transmises.

9. Procédé selon la revendication 8, dans lequel les champs magnétiques sont générés par au moins un ensemble de trois bobines mutuellement perpendiculaires (10a', 10a", 10a"').

10. Procédé selon l'une quelconque des revendications 8 ou 9, dans lequel l'unité magnétique (44) inclut une source d'alimentation qui est utilisée comme source de tension pour des éléments dans le dispositif de détection in vivo (40).

11. Procédé selon la revendication 10, dans lequel la position du dispositif in vivo est déterminée à partir d'une puissance induite dans chaque bobine par les champs magnétiques.

12. Procédé selon la revendication 9 ou 10, comprenant le fait d'amener alternativement les une ou plusieurs bobines conductrices (49) à détecter des champs magnétiques et à fournir une tension à des unités dans le dispositif de détection in vivo (40).

13. Procédé selon l'une quelconque des revendications 8 à 12, dans lequel les champs magnétiques générés par le générateur de champs magnétiques sont générés à des instants différents.

14. Procédé selon l'une quelconque des revendications 8 à 13, dans lequel les champs magnétiques générés par le générateur de champs magnétiques sont générés simultanément avec des fréquences différentes.
